# EUROPEAN PATENT APPLICATION

(11) **EP 4 759 224 A1**
(43) Date of publication of application: **17.06.2026**
(21) Application number: 24383346.4
(22) Date of filing: 10.12.2024
(51) Int. Cl.: A61B 5/02, A61B 5/026, A61B 5/00

(54) **A COMPUTER-IMPLEMENTED METHOD, A SYSTEM AND A COMPUTER PROGRAM FOR DETERMINING A GLOBAL CARDIOVASCULAR/HEMODYNAMIC PARAMETER OF A SUBJECT**

(71) Applicant: Fundació Institut de Ciències Fotòniques, 08860 Castelldefels (Barcelona) (ES); Institució Catalana de Recerca i Estudis Avançats, 08010 Barcelona (ES); Fundació Institut d'Investigació i Innovació Parc Taulí (I3PT), 08208 Sabadell (Barcelona) (ES)
(72) Inventor: DURDURAN, Turgut, 08860 CASTELLDEFELS (ES); ZANOLETTI, Marta, 08860 CASTELLDEFELS (ES); CORTESE, Lorenzo, 08860 CASTELLDEFELS (ES); YAQUB, Muhammad Atif, 08860 CASTELLDEFELS (ES); MESQUIDA, Jaume, 08208 SABADELL (ES)
(74) Representative: Ponti & Partners, S.L.P

(57) **Abstract**

The present invention relates to a computer-implemented method for determining a global cardiovascular/hemodynamic parameter of a subject, comprising:
a) receiving data representative of non-invasive and optical measurements of microvascular local blood flow of a muscle or tissue of a current subject, previously acquired by optical techniques based on laser speckle statistics, wherein that muscle or tissue is in a non-compartmentalized area and has a limited autoregulatory performance where the local blood flow cannot be isolated from that of the systemic one;
b) processing the data received at a);
c) determining the global cardiovascular/hemodynamic parameter of the current subject or/and an index originating or representative of the global cardiovascular/hemodynamic parameter, based on the result of step b); and
d) providing the determined global cardiovascular/hemodynamic parameter or/and the determined index.

The present invention also relates to a system and a computer program implementing the method of the invention.

## Description

### FIELD OF THE INVENTION

The present invention relates, in a first aspect, to a computer-implemented method for determining a global cardiovascular/hemodynamic parameter of a subject.

A second aspect of the present invention relates to a system adapted to implement the method of the first aspect of the invention.

A third aspect of the present invention relates to a computer program adapted to implement the method of the first aspect of the invention.

### BACKGROUND OF THE INVENTION

There are different proposals known in the art for determining a global cardiovascular/hemodynamic parameter of a subject, particularly for determining Cardiac Output (CO).

Specifically, currently, several technologies can estimate CO value. They might be divided into invasive, minimally invasive, and non-invasive, depending on the need and location of catheters and/or probes entering the human body:
- Invasive technologies:
   · Pulmonary artery catheter (PAC) with transcardiac thermodilution (also referred to as Swan-Ganz)
- Minimally invasive or semi-invasive technologies:
   · Pulse waveform analysis (PWA) through an arterial catheter (usually on the wrist or the groin).
      - Calibrated: with Transpulmonary thermodilution (TPTD).
      - Non-calibrated (or internally calibrated).
   · Oesophageal doppler
- Non-invasive technologies:
   · Bioreactance/bioimpedance.
   · Tonometry/Volume clamp method.

Regrettably, non-invasive technologies lack reliability, especially in critical conditions, and they are not recommended for clinical use in settings such as the intensive care unit (ICU) or the Operating Room (OR), where the severity or the risks are of utmost importance.

In critical care settings, the most widely used technologies are PWA with TPTD calibration, and the PAC. The accuracy of both technologies is similar, and the election of one or the other will depend on the baseline profile of the patient, prioritizing the PAC in those patients with previous severe cardiac conditions or ARDS. In the operating room, internally calibrated PWA technologies are widely implemented. This fact derives from the reliability of such technologies to track CO changes in conditions where vasoplegia does not play a major role, that is, where vascular tone does not vary from beat-to-beat, as in blood loss situations as results of the surgery. Of note, in emergent surgeries of septic patients, internally calibrated PWA systems are prone to significant reliability issues, deriving in inaccuracy when guiding fluid administration. Likewise, non-invasive technologies are only recommended for use in low-risk patients and procedures.

It is, therefore, necessary to provide an alternative to the state of the art by providing a method, system and computer program for determining a global cardiovascular/hemodynamic parameter of a subject which do not possess the above mentioned drawbacks of those of the prior art.

### SUMMARY OF THE INVENTION

To that end, the present invention relates, in a first aspect, to a computer-implemented method for determining a global cardiovascular/hemodynamic parameter of a subject, comprising:
a) receiving data representative of non-invasive and optical measurements of microvascular local blood flow of a muscle or tissue (such as a skeletal muscle) of a current subject, previously acquired by optical techniques based on laser speckle statistics, wherein said muscle or tissue is in a non-compartmentalized area and has a limited autoregulatory performance where the local blood flow cannot be well isolated from that of the systemic one;
b) processing said data received at a);
c) determining said global cardiovascular/hemodynamic parameter of said current subject or/and an index originating or representative of the global cardiovascular/hemodynamic parameter, based on the result of step b); and
d) providing said determined global cardiovascular/hemodynamic parameter or/and said determined index.

For implementing the present invention, data coming from optical probes placed on different locations, such as the subject's arms, legs, torso and so forth, can be used. The rationale for using a muscle or tissue located in a non-compartmentalized area and having a limited autoregulatory performance, for deriving the global cardiovascular/hemodynamic parameter, such as CO values, comes from two main key points: (1) It lacks strong autoregulatory protective mechanisms; and (2) it is an "open" compartment.
(1) Autoregulation allows for maintaining a constant or sufficient regional blood flow in accordance with local metabolic demands, and independently of global cardiovascular changes. Therefore, in those organs with strong autoregulatory capabilities, such as the brain or the heart, the correlation between local blood flow and CO will be highly variable depending on the local metabolic demands. Although this physiological mechanism allows for the protection of vital areas even in conditions of very low CO, it is a limitation when trying to infer CO from this local blood flow measurement. On the other hand, in muscles or tissues with limited autoregulatory performance, such as the skeletal muscle, local blood flow and its changes will be highly linked to global blood flow upon similar local metabolic demand. This true for determining global cardiovascular/hemodynamic parameters other than CO. Therefore, limited auto-regulatory performance refers to the limited ability of such muscles or tissues to adapt their flow to changes in blood pressure (drop in pressure will lead to decreased flow), as compared to other vital areas (such as the heart or the brain), where blood flow is kept mostly constant despite changes in blood pressure. In other words, commonly auto-regulation is defined as the correlation between the measurement of blood flow by Doppler Ultrasound on the target tissue. Organs with good autoregulatory performance can isolate the local blood flow from that of the systemic one. This is typically quantified as a number between -1 to 1 derived from some form of (often a "Pearson's") correlation coefficient. When this parameter is negative or below a certain threshold (e.g. 0.6), the brain is said to be in a healthy or safe condition in terms of autoregulation. If this raises above that, then corrective actions are suggested. A similar threshold for the peripheral muscle is not yet known but it is assumed that for the healthy muscles the coefficient is higher, even a perfect correlation [22,23,24,25].
(2) In the absence of direct damage to the tissue, the above mentioned muscle or tissue is not affected by local compartment issues, as occurs with compartmentalized areas, such as the brain or the splanchnic territory. In "closed" compartments, blood flow may be impaired by increased pressure within the compartment. This happens, for instance, in different brain injuries, where increased intracranial pressure serves as an opposing force to blood flow. That is also true for the splanchnic territory, in different abdominal conditions causing increased intraabdominal pressure. Since these pathological situations are highly prevalent in critical care patients, estimating CO from local blood flow measurements from these compartmentalized areas may not be accurate, and even linked to profound variations within the same subject as the disease evolves. This is also true for determining global cardiovascular/hemodynamic parameters other than CO.

Non-limitative examples of muscles or tissues located in a non-compartmentalized area and with limited autoregulatory performance, which can be used according to the present invention, i.e. that meet the above two main key points, are: skeletal muscles, adipose tissue (like breast tissue, which is very accessible), earlobes, cheeks, sternum, etc.

In the present invention, the term global cardiovascular/hemodynamic parameter has to be understood as any parameter informing on global blood flow (such as cardiac output and/or stroke volume), and on vascular tone (such as obtained by means of estimating arterial elastane and/or arterial impedance), or a parameter proportional thereto.

Depending on the embodiment, the global cardiovascular/hemodynamic parameter is cardiac output, stroke volume, and vascular tone (such as obtained by means of estimating arterial elastane and/or arterial impedance).

The above mentioned optical techniques based on laser speckle statistics is any known in the art considered appropriate for the present invention, such as diffuse correlation spectroscopy (DCS), diffuse wave spectroscopy (DWS), near-infrared spectroscopy (NIRS), speckle contrast optical spectroscopy (SCOS), laser speckle contrast spectroscopy/tomography/imaging, interference based techniques (iNIRS/iDCS), and/or derivatives of those techniques. The measurements reflect the status of the hemodynamics of the microvasculature (including, but not limited to, arterioles, capillaries and venules) from the skin to a depth of several centimetres, particularly up to three centimetres deep.

In the present invention, the term index must be interpreted in a broad manner, so that it refers to any type of element that when provided at step d) indicates, whether numerically, graphically, textually, visually, audibly, etc., a value or values representative of the global cardiovascular/hemodynamic parameter, or, when not provided at step d) but only used for obtaining the global cardiovascular/hemodynamic parameter at step c), i.e. when originating that parameter, refers to a signal, such as a data signal including a value or values used for determining the global cardiovascular/hemodynamic parameter.

In addition, in the present invention, step d) comprises providing the determined global cardiovascular/hemodynamic parameter or/and determined index in any possible manner, whether to a user, by means of an appropriate device (display, speaker, haptic device, etc.) and/or to a system/machine.

For an embodiment, the determined index is a microvascular blood flow index (BFI).

For an embodiment, steps b) and c) are performed with the proviso that no physiological data of at least the current subject are used.

According to an embodiment, steps b) and c) are performed with the proviso that no further measurements of at least the current subject are used, apart from the above mentioned non-invasive and optical microvascular measurements of microvascular local blood flow of the above mentioned muscle or tissue of step a).

According to an embodiment, at least steps b) and c) are performed with an artificial intelligence (Al) learning algorithm trained with at least one training dataset of data of signals representative of non-invasive optical measurements of at least local blood flow of a plurality of previous subjects, wherein the global cardiovascular/hemodynamic parameter of the current subject or/and the above mentioned index is/are determined by estimating its value, or a transient increase and/or decrease thereof, or no change thereof, with a mathematical estimator learned with that artificial intelligence learning algorithm.

Thus, for this embodiment, in the present invention, the "previous subjects" refer to those subjects from which training data is obtained and used to develop the AI learning algorithm, while the "current subject" refer to the subject benefiting from the already trained AI learning algorithm. A similar meaning for those terms is given for embodiments not including an AI learning algorithm.

For an embodiment, the artificial intelligence algorithm is a machine learning algorithm. For an implementation of that embodiment, the machine learning algorithm is a deep learning algorithm.

For variants of that implementation, the deep learning algorithm implements at least one of a convolutional neural network (CNN), a recurrent neural network (RNN), and a hybrid RNN/CNN, and/or implement strategies such as long short-term memory units, gated recurrent units to overcome typical problems of standard RNN. Ensemble methods and autoencoders can also enhance predictions, and are thus used by the method of the present invention, for some embodiment.

Nontemporal-neural network models like classical time-series forecasting methods (e.g., ARIMA), decision trees, random forests, gradient boosting machines, and support vector machines (SVM) etc. can also be used, for some embodiments.

For an implementation of that embodiment, the at least one training dataset comprises at least one training dataset including data of signal features of pulsatile signals representative of non-invasive and optical measurements of pulsatile microvascular local blood flow of muscles or tissues of a plurality of previous subjects, previously acquired by optical techniques based on laser speckle statistics, wherein said muscles or tissues are located in non-compartmentalized areas and have a limited autoregulatory performance where the local blood flow cannot be isolated from that of the systemic one, and wherein the pulsatility of said pulsatile signals corresponds to the cardiac cycle.

For variants of that implementation said signal features include one or more of following features corresponding to systole and diastole periods of the pulsatile signals: maximum and minimum, an area under the curve, a second peak, and a dicrotic point; and/or one or more of the following temporal features: time from the foot to the peak of the pulsatile signal (systolic time); time from the peak to the foot of the next pulsatile signals (diastolic time); temporal width of said pulsatile signal; cycle duration; upstroke time; peak to notch time.

For an embodiment, the above mentioned signal features are extracted and/or learned from signals including at least two different time periods.

For different implementations of that embodiment, those two different time periods include changes in the pulsatile signals of the previous subjects, caused through a fluid management manoeuvre/challenge on the corresponding previous subject, such as through a Passive Leg Raising (PLR) protocol manoeuvre, administration of intravenous fluid bolus, of inotropic agents, etc.

Thus, for those implementations, the determination of the global cardiovascular/hemodynamic parameter is inferred from changes caused by the application of a fluid management manoeuvre/challenge on the current subject/patient and acquiring speckle data before and after that application.

According to an embodiment, at least said steps b) and c) are performed with an artificial intelligence learning algorithm trained with at least one training dataset of data of signals representative of non-invasive and/or invasive measurements not based on laser speckle statistics of a plurality of previous subjects, wherein the global cardiovascular/hemodynamic parameter of the current subject or/and said index is/are determined by estimating its value, or a transient increase and/or decrease thereof, or no change thereof, with a mathematical estimator learned with said artificial intelligence learning algorithm.

For an embodiment, the above mentioned at least one training dataset further comprises a training dataset including data of at least the global cardiovascular/hemodynamic parameter, for the plurality of previous subjects, obtained from measurements not based on laser speckle statistics but synchronized, for each previous subject, with the non-invasive and optical measurements of pulsatile microvascular local blood flow of a muscle or tissue of the corresponding previous subject, wherein said muscle or tissue is located in a non-compartmentalized area and has a limited autoregulatory performance where the local blood flow cannot be isolated from that of the systemic one.

According to an embodiment, the at least one training dataset further comprises a training dataset including at least one of the following data related to the plurality of previous subjects: demographic data, anatomical data, pharmacotherapy data, and augmented data.

The computer-implemented method of the first aspect of the present invention further comprises, for an embodiment, training the above mentioned artificial intelligence learning algorithm with the at least one training dataset.

According to an implementation of that embodiment, the method further comprises, prior to said training, for the plurality of previous subjects:
- a data acquisition step comprising receiving acquired laser speckle statistics and, synchronized for the same previous subject, the above mentioned data of at least the global cardiovascular/hemodynamic parameter obtained from measurements not based on laser speckle statistics;
- pre-processing said acquired laser speckle statistics to derive pulsatile signals representative of pulsatile blood flow; and
- filtering the derived pulsatile signals to obtain the pulsatile signals of the at least one training dataset.

According to an embodiment, at least steps b) and c) are performed with a logistic-based regressor used to fit an inputted set of extracted features included in the data received at a) to output the global cardiovascular/hemodynamic parameter or/and the index originating or representative of the global cardiovascular/hemodynamic parameter.*

For an implementation of that embodiment, a first input formed by pulsatile BFI is provided to the logistic-based regressor. Since BFI is pulsatile, some or all of the above mentioned signal features are extracted from the time trace, as well as average values, calculated before intervention, during intervention and after intervention.

For another implementation, a further input formed by time CO time traces is provided to the logistic-based regressor. In this case, CO time trace would be used to create classes such as (but not limited to):
Example 1 (dichotomization):
   - Responder (CO>10%)
   - Non-responder (CO<10%)
Example 2 (levels):
   - Unchanged (delta CO =0%)
   - Mild change (delta CO > 0 % and less 10%)
   - Change (delta CO >10%)

The starting point for the logistic-base regressor embodiment would be still the time traces. This cover more a supervised machine learning method for classification and not just prediction of absolute values and trends.

For an embodiment, the computer-implemented method of the first aspect of the invention and/or a method comprising the same further comprises, before step a), a previous step for performing the non-invasive and optical measurements of pulsatile microvascular local blood flow on the muscle or tissue of the current subject, wherein said muscle or tissue is located in a non-compartmentalized area and has a limited autoregulatory performance where the local blood flow cannot be isolated from that of the systemic one.

A second aspect of the present invention relates to a system for determining a global cardiovascular/hemodynamic parameter of a subject, comprising a processing device including a processor and a computer-readable medium having encoded thereon computer-executable instructions to cause the processor to execute the computer-implemented method of the first aspect of the present invention.

A third aspect of the present invention relates to a computer program, including code instructions that when executed on at least one processor perform the steps of the computer-implemented method of the first aspect of the present invention.

A computer program product is further provided by the present invention, comprising a tangible medium and, stored therein, the computer program of the third aspect of the present invention.

The present invention will have an impact on two of the major issues in the resuscitation process of acutely ill patients: determining the relationship between preload (fluid administration) and cardiac performance as well as to perform cardiac output monitoring and/or other global cardiovascular/hemodynamic parameters.

An optically-guided fluid therapy management based on cardiac output changes is implemented for some embodiments of the present invention and/or for further method/system aspects of the present invention, which will be further described below in a later section herein.

### BRIEF DESCRIPTION OF FIGURES

In the following some preferred embodiments of the invention will be described with reference to the enclosed figures. They are provided only for illustration purposes without however limiting the scope of the invention. In accordance with common practice, the components in Figures are drawn to emphasize specific features and they are not drawn to the right scale.
Figure 1A is a plot showing an example of averaged pulses in two different time periods of the PLR protocol of a pulsatile signal representative of non-invasive and optical measurements of pulsatile microvascular local blood flow of skeletal muscles of one of a plurality of previous subjects, previously acquired by optical techniques based on laser speckle statistics. Some signal features are indicated in the plot and included in a training dataset of the method of the first aspect of the invention, for some embodiments.
Figure 1B is a plot showing an example of some signal features indicated in the plot and included in a training dataset of the method of the first aspect of the invention, for some embodiments. The illustrated plot is only schematic and used to identify those signal features, the latter being learned/extracted from the plot of Figure 1A, i.e. of averaged pulses in two different time periods of the PLR protocol, for an embodiment.
Figure 2 is a scheme of the process to derive CO from speckle statistics of a current subject and non-invasive CO acquisition of a plurality of previous subjects, according to the method of the first aspect of the present invention, for an embodiment.
Figure 3 is a plot showing the Frank-Starling law of the heart.
Figure 4 is a schematic diagram of the system of the second aspect of the present invention, for an embodiment for which the system implements an optically-guided fluid therapy management application.
Figure 5 is a scheme of the PLR conducted in a proof-of-concept study to support the implementation of the present invention, for an embodiment.
Figure 6 is a schematic diagram showing CO measured by means of a macrocirculation hemodynamic monitor and BFI measured by a diffuse optical device synchronized and re-sampled at 10 Hz, which are used as an input of an unsupervised model to predict the CO looking at the BFI feature, for an embodiment of the present invention.
Figure 7A is a plot showing an example of a fast relative BFI (rBFI), where the pulsatile component is present.
Figure 7B illustrates by means of 24 plots the fast relative BFI of each of the 24 healthy subjects of the proof-of-concept study of Figure 5.
Figure 8 include some plots as examples of two non-responder subjects (panel A and C) and two responder subjects (panel B and D) of the proof-of-concept study of Figure 5. The measured CO is reported in solid circles, while the prediction by solid triangles every 30 samples.
Figure 9 illustrates results obtained when applying the present invention to different patients, for some embodiments, for estimating the BFI and rBFI indexes in unhealthy subjects/patients. Four different patients: two septic patients with high/low dosage of NE and two non-septic patients with high/low dosage of NE. NE: norepinephrine in mcg/kg/min; Peak_f: peak frequency (heart beat in beats per minute, bpm); SNR: signal to noise ratio.

### DESCRIPTION OF THE PREFERRED EMBODIMENTS

Different embodiments of the present invention will be described in the present section with reference to the appended Figures.

As already disclosed in a previous section, for some embodiments of the method of the first aspect of the present invention, at least steps b) and c) are performed with an artificial intelligence learning algorithm trained with one or more training datasets.

In the present section, those possible training datasets are described below, for some embodiments, and respectively grouped in different inputs for training the artificial intelligence learning algorithm.

### Input 1 for training - Pulsatile signal derived from speckle statistics (temporal/spatial):

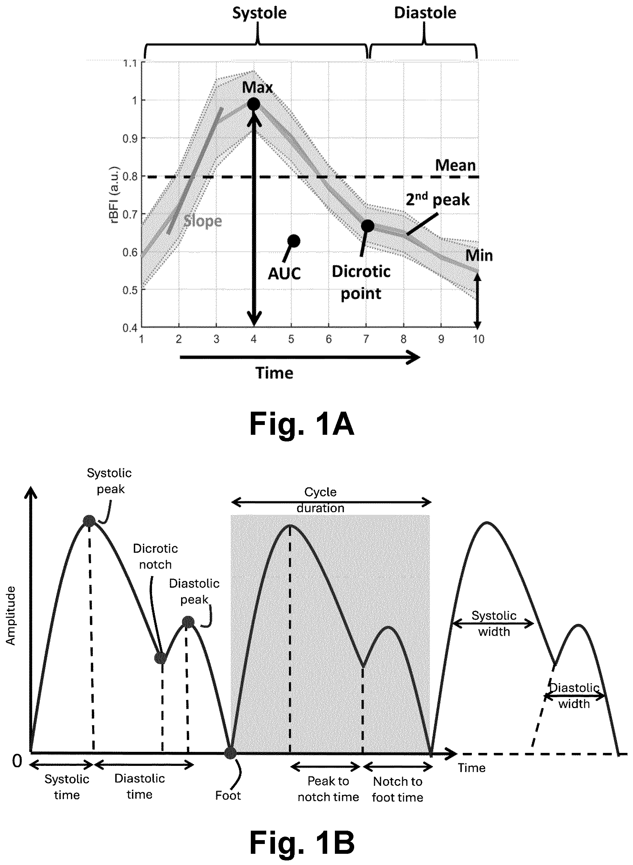

For an embodiment, the AI learning algorithm implements a hybrid recurrent and convolutional neuronal network trained with a training dataset (Input 1) including data of the above mentioned signal features, such as the one shown in Figures 1A and 1B.

Those signal features of pulsatile signals are representative of non-invasive and optical measurements of pulsatile microvascular local blood flow of skeletal muscles of a plurality of previous subjects, previously acquired by optical techniques based on laser speckle statistics obtained from a non-invasive speckle based method such as diffuse correlation spectroscopy, diffuse wave spectroscopy, laser speckle contrast spectroscopy/tomography/imaging, interference based techniques (iNIRS/iDCS), etc.

Figure 1A shows some main signal features during the systole and diastole periods: maximum (max) and minimum (min), corresponding to the end of diastole period, the area under the curve (AUC), the 2nd peak and the dicrotic point.

Similarly, Figure 1B shows further main signal features, including temporal features, of the pulsatile signal:
- Systolic peak (highest blood pressure in the arteries) represents the peak force the heart generates to push blood through the circulatory system.
- Diastolic peak (lowest blood pressure in the arteries) occurs when the heart is at rest between beats (during diastole), allowing the chambers to refill with blood.
- Dicrotic notch is caused by the brief backflow of blood as the aortic valve closes. It marks the transition between systole and diastole.
- Cycle duration refers to the cardiac cycle and it includes both systole and diastole. Typically, it is around 0.8 s in healthy adults.
- Systolic time (systolic upstroke time) refers to the interval between the beginning of the cardiac cycle (start of the ventricular contraction) and the systolic peak.
- Diastolic time refers to the period when the heart is in diastole (relaxation) and the ventricles are filling with blood.
- Systolic width reflects the period when the heart is contracting and ejecting blood into the arteries.
- Diastolic width reflects the time the heart spends relaxing and refilling with blood.
- Peak to notch time reflects the transition from systole to diastole.
- Notch to foot time reflects the transition from diastole to the next cardiac cycle systolic phase.
- Foot marks the onset of the systolic phase.

Some or all of the above listed signal features for the input 1 for training the AI learning algorithm, depending on the embodiment.

### Input 2 for training - cardiac output:

A further training dataset, input 2, is used, for some embodiments, for training the AI learning algorithm, and comprises data of signals representative of non-invasive and/or invasive measurements/methods not based on laser speckle statistics of a plurality of previous subjects, such as the ones listed below.
- Non-invasive methods [3,4] - used mainly in routine measurements:
   ∘ CO estimation from the volume clamp method using a non-invasive monitor (such as Finapress^{®}).
   ∘ Echocardiography [5].: uses ultrasound to create images of the heart, measuring stroke volume and heart rate (CO = SV x HR).
   ∘ Bioimpedance [6-8]: electrodes are placed on the chest to measure the electrical impedance caused by blood flow. A refinement of this technique is called bioreactance and utilizes the phase shifts in the electrical current.
   ∘ Doppler ultrasound [9]: measures blood flow velocity in large vessels combined with vessels diameter. A transcutaneous implementation is also possible.
   ∘ Pulse wave analysis [10,11]: uses peripheral arterial waveform (from finger/wrist cuff).
   ∘ Cardiac MRI [12] can measure blood flow and heart function.
   ∘ Photoplethysmography [13] - uses light (not speckles) but apparently is not interchangeable and needs to be utilized with care.
- Invasive methods - often used in critically ill patients where continuous measurements are necessary:
   ∘ Pulmonary artery catheterization (PAC) [1], based on catheter insertion into a large vein (jugular, femoral) up to the pulmonary artery. Two different techniques are used to calculate CO: 1) thermodilution, where a cold saline solution is injected through the catheter or 2) direct Fick method, where oxygen consumption and arterial-venous oxygen difference is measured.
   ∘ Intra-arterial BP measurement with pulse contour analysis [2], where a catheter is inserted into the artery and CO is estimated by analysing the arterial waveform.

Besides the above methods for estimating CO, there are other methods of derivation CO by means of techniques to estimate mean arterial pressure (MAP) and compliance. Measurements obtained with these other methods are included in the input 2 training dataset, for some embodiments.

Examples of those methods of extracting MAP and compliance are provided below.

For mean arterial pressure:
- Invasive methods:
   ∘ Arterial catheterization: an arterial line is inserted into radial or femoral artery providing continuous and accurate MAP.
- Non-invasive:
   ∘ Automated blood pressure cuff [14-16], with oscillometric methods (estimate systolic and diastolic blood pressure).
   ∘ Tonometry [16,17]: a device is placed over a superficial artery (e.g. radial artery) to measure pressure waveforms.
   ∘ Finger cuffs: devices like the ones used in this invention disclosure (Finapres^{®}) or others (Nexfin^{®}) use a cuff around the finger (clamp volume method [18,19]).

For compliance [19]:
- Pulse wave velocity [20]: measures the speed at which the blood pressure travel through the arteries (sensors are placed at two points along major arteries).
- Pulse contour analysis: analyse the shape of the arterial pressure waveform (invasive or non-invasive).
- Arterial and applanation tonometry: sensor flattens the arteries to measure pressure waves directly.
- Echocardiography: uses ultrasound to measure diameter of major arteries. The change in the diameter provide an estimate of arterial compliance.
- MRI: measure arterial dimensions and blood flow.

### Further Input for training:

For some embodiments, diversity and robustness of the data set is improved by including a further training dataset for training the AI learning algorithm, including input data of the plurality of previous subjects, such as the input data listed below.
- Demographic data: biological sex, age, BMI,...
- Anatomical data: adipose tissue thickness which affects the optical data.
- Pharmacotherapy: use of drugs affecting the vascular tone or the pulsatile signal, such as norepinephrine, vasopressin, or sedative agents.
- Data augmentation:
   ∘ Utilizing the same data set by time wrapping, jittering, shifting to increase diversity.
   ∘ Increase the sample size including critically ill patients and different methods of CO sensing.
   ∘ Other interventions to increase cardiac output [21].

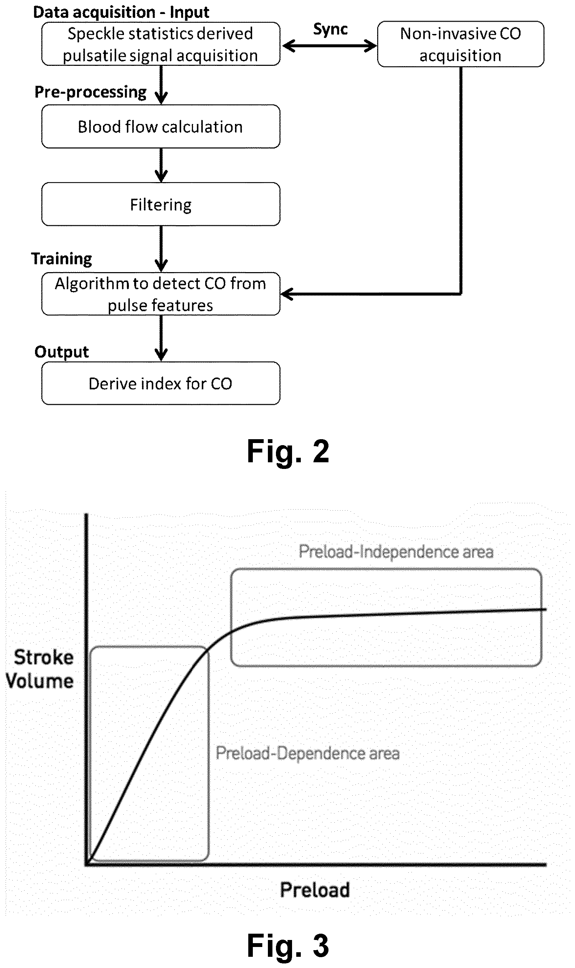

As shown in Figure 2, according to an embodiment, the method further comprises, prior to the training of the AI learning algorithm, for the plurality of previous subjects, the following steps (included in the two upper blocks of Figure 2):
- A data acquisition step, "Data acquisition - Input" in Figure 2, comprising: receiving acquired laser speckle statistics and, synchronized for the same previous subject, the above mentioned data of at least the global cardiovascular/hemodynamic parameter obtained from measurements not based on laser speckle statistics (non-invasive CO acquisition in Figure 2);
- A pre-processing step comprising pre-processing those acquired laser speckle statistics to derive pulsatile signals representative of pulsatile blood flow; and
- A filtering step comprising filtering the derived pulsatile signals to obtain the pulsatile signals of the at least one training dataset.

Figure 2 also schematically shows, in its two lower block, the above described steps of the method of the first aspect of the invention related to the training of the Al learning algorithm and the outputting of the index originating the CO once estimated by the Al learning algorithm from the data received at step a) representative of non-invasive and optical measurements of microvascular local blood flow of a skeletal muscle of the current subject.

For an embodiment, known CO values are also inputted in the Al learning algorithm as ground-truth values, and, subsequently, the algorithm is evaluated using data from a subject not included in the training phase.

As stated in a previous section, depending on the embodiment of the method of the first aspect of the present invention, for predicting cardiac output from pulsatile signals, besides hybrid recurrent neural networks (RNN) it is possible to implement strategies such as long short-term memory units, gated recurrent units to overcome typical problems of standard RNN. Ensemble methods and autoencoders can also enhance predictions. Nontemporal-neural network models like classical time-series forecasting methods (e.g., ARIMA), decision trees, random forests, gradient boosting machines, and support vector machines (SVM) etc. can also be used.

With the present invention, CO (and similarly other global cardiovascular/hemodynamic parameter value) are estimated from a non-invasive measurement on local muscle or tissue. This estimation allows for accurately tracking CO changes when performing hemodynamic interventions, either the administration of fluids, or predictive manoeuvres, such as the passive leg raising test (PLR). These predictive manoeuvres challenge the heart, testing whether CO would increase as results of the infusion of fluids (increasing cardiac preload), guiding the optimization of cardiac performance while avoiding the deleterious administration of fluids, which is crucial in daily practice in acute care.

It will be also possible to infer vascular tone as swell as responsiveness and non-responsiveness to fluid management.

Thus, as stated in a previous section, an optically-guided fluid therapy management based on cardiac output changes is implemented for some embodiments of the present invention and/or for further method/system aspects of the present invention, which will be further described below.

### Optically-guided fluid therapy management:

Cardiac output (CO) equals stroke volume (SV) of the left ventricle times the heart rate (HR). Whereas the technologies of the prior art derive CO and SV through temperature and pressure measurements, the present invention allows estimating global blood flow (and therefore SV and CO) directly from measurements of the microcirculatory local blood flow in muscles or tissues, particularly in skeletal muscles, like arms or legs, using laser speckle statistics-based techniques such as diffuse correlation spectroscopy (DCS), and in particular from the pulsatile component of the blood flow.

Since only part of the CO will be derived to the explored local area measured, making additional anthropometric corrections (age, gender, body weight, height, forearm circumference, fat layer), for some embodiments of all the aspects of the present invention, will allow estimating more accurate SV values. Likewise, in the PWA technologies, the area under the curve (AUC) of the pulsatile signal will correspond to a given SV value, allowing for a beat-to-beat analysis and providing reliable information on SV and CO changes over time. Unlike in the PWA technologies, where the AUC derives from the interaction between stroke volume and vascular tone, the pulsatile signal obtained with DCS is a pure flow measurement, and therefore, it will allow overcoming one of the major limitations of PWA technologies, which is their lack of reliability in situations where vascular tone is impaired or changing, such as after vasopressor support modifications. Of note, these situations are extremely usual in daily critical care, deriving in a recommendation of avoiding non-calibrated or internally calibrated devices, and the recommendation of re-calibrating the PWA signal with thermodilution measurements after performing interventions (best practice recommendation), or every 4-8 hours (manufacturer recommendation).

### Daily clinical practice:

In acute care, along with the etiological treatment of the underlying disease, supportive therapies are provided aiming at restoring and maintaining the adequacy of tissue oxygenation, in order to avoid cellular hypoxia and, consequently, preventing the development of multiple organ failure and the eventual death of the individual. The oxygen delivered to the tissues depends on two systems that are intimately related: (i) the respiratory, and (ii) the cardiovascular system. While respiratory support targets the consecution of certain values of arterial oxygenation (PaO2), cardiovascular support aims at increasing the global blood flow for delivering that arterial oxygen content to the tissues to meet their metabolic demands. Therefore, cardiovascular support pivots on the monitoring and manipulation of global blood flow, represented by CO.

Depending on the underlying cardiac performance of the individual, the intervention required for increasing CO might be completely different. Except for some exceptional cases of bradycardia, manipulation of CO is driven by manipulation of the SV. Based on the Frank-Starling approach to cardiac performance, that considers the relationship between cardiac preload (fluid management therapy) and a given myocardial contractility, The present inventors can define two different situations: (A) preload-dependence and (B) preload-independence (Figure 3). In the first situation, the SV will increase as results of increasing cardiac preload (venous return), while in the second situation, changes in preload will not result in increases in SV, and might only associate detrimental effects, such as pulmonary edema. Consequently, the chosen intervention for increasing SV when the heart is working in preload-dependence conditions will be the administration of intravenous fluids. On the other hand, when the heart is working in a preload-independent area, the increase in SV will be only achieved after modifying cardiac contractility, by means of inotropic agents.

This physiological approach to CO manipulation is made independently of the severity of the disease, and of the area where the patient is treated, such as the emergency department (ED), the ICU, or the OR. However, the degree of invasiveness and monitoring will be directly related to all these determinants. Hence, a less severe patient arriving to the ED will not be invasively monitored unless a negative evolution or depending on the risk-benefit of a less invasive vs a more invasive management. For instance, a complex patient with a previous heart condition might be more invasively and closely monitored despite a less severe acute condition, in order to avoid fatal negative effects of a "blind" cardiovascular/hemodynamic resuscitation (see paragraph below) with non-monitored fluid administration potentially leading to pulmonary edema.

Generally, fluid administration is considered the first step in the resuscitation process. Fluid expansion aims at increasing venous return, cardiac preload and, if the patient is in the preload-responsive area of the Frank-Starling curve of cardiac performance, ultimately CO and oxygen delivery to the tissues (DO₂: Delivered Oxygen). This is of utmost importance, since hemodynamic benefits would only be expected when the patient is situated in the fluid-responsive area; otherwise fluid will only be detrimental. Hence, this preload-dependency index is highly recommended for guiding fluid resuscitation in acute care settings. A robust body of evidence shows that fluid overload is associated with increased morbidity and mortality, particularly in septic patients. Therefore, current approach to fluid administration relies fundamentally on the conceptual framework of the Frank-Starling curve of cardiac performance. This approach is still considered the gold standard for evaluating fluid responsiveness at the bedside. However, this approach requires an invasive arterial catheter and implies that the effect of volume expansion will be only evaluated once the volume has been administered. Unfortunately, since in shock states fluid-responsiveness might account for approximately 50% of the cases, no positive benefits are expected in half of the fluid challenges. Of note, fluid-responsiveness will vary in the time course of the underlying disease, and fluid-responsive patients might rapidly become non-responders, and in aggressive fluid loading scenarios, downstream harmful effects will largely overcome the minimal impact on stroke volume. Therefore, the use of bedside tools to monitor and/or predict the hemodynamic effect of a fluid bolus prior to its administration is mandatory, especially in more complex or severe scenarios. The present invention, coupled for instance to a passive leg raising (PLR) manoeuvre to produce a transient and conservative increase in CO, represents a non-invasive and real-time predictor for fluid responsiveness prior to any potentially harmful intervention.

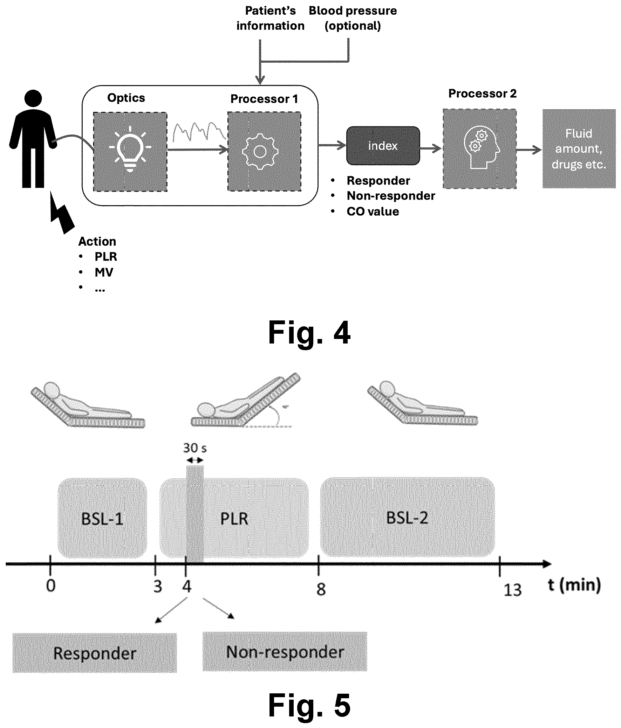

A schematic diagram of the system of the second aspect of the present invention is shown in Figure 4, for an embodiment implementing the here described optically-guided fluid therapy management application. The diagram also indicates the different actions/operations performed by the system or steps performed by the method of the first aspect.

As shown in Figure 4, following an action (PLR: passive leg raising, MV: mechanical ventilation), the acquisition (by means of block "Optics") of optically derived parameters will be processed together with patient's information ("where "patient" refers to the above called current subject) and optionally together with blood pressure (also of the current subject). The process (implemented by "processor 1", for the illustrated embodiment) will predict an index that it can be the type of response to the action (responder and non-responder) as well as a cardiac output value (CO: cardiac output), or an index originating that CO value (or other global cardiovascular/hemodynamic parameter). These responses will be fed to a processor 2 (either automatically or by clinicians) which will decide on fluid amounts, drugs to use etc. Processor 1 and 2 could be the part of the same processing entity or separate processors, depending on the embodiment.

In the following, some proof-of-concepts studies and data analysis that support the implementation of the present invention, will be described.

### A) Proof-of-concept experiments on the link between macrocirculation (upstream) and microcirculation (downstream):

The present inventors performed a PLR maneuver on 24 healthy subjects. The protocol consists in three minutes of baseline, five minutes of passive leg rising and five minutes rest. A schematic of the protocol is shown in Figure 5. A subject is defined "responder" or "non-responder" according to the changes in the cardiac output (CO, in L/min) measured by a commercially available non-invasive hemodynamic monitor, 60 seconds after PLR period. A subject is "responder" when CO changes at least of 15% in the PLR with respect to the baseline. The local microcirculation (on the brachioradialis muscle is measured by means of diffuse optical method (Durduran, et al. 2010, 73: 076701). The macrocirculation hemodynamic monitor and local microcirculation monitor are synchronized via hardware by means of a 10 Hz transit-to-logic signal.

In this proof-of-concept study, the present inventors used a volume-clamp method to estimate CO (Finapres^{®}, Finapres Medical Systems). The use of a non-invasive hemodynamic monitor as a "ground-truth" is acceptable in a healthy population for the following reasons: 1) invasive monitoring is not advisable/possible; 2) agreement between pulse-contour analysis of the volume-clamp system and US or invasively obtained CO values is acceptable in certain populations, such as outpatient populations (Van der Spoel AGE et al. J Clin Anesth 2012; 24:304-309) or post-cardiac surgery stable patients (Bogert LWJ et al, Anaesthesia 2010; 65:1119-1125); and 3) the vascular tone is not compromised in these subjects, avoiding the main limitation of the non-invasive technologies, which derives from vascular tone alterations and/or peripheral hypoperfusion in unstable conditions. In fact, the present inventors also monitored the vascular tone of the healthy subjects included in this study by means of the perfusion index obtained by the pulse oximeter integrated into the optical device. The subjects included showed a perfusion index of 2.4 ± 0.4 %, which is within the normal range.

The present inventors then obtained synchronized cardiac output (CO, in L/min) and microvascular blood flow index (BFI, in cm²/s). CO and BFI are sampled at the common frequency of 10 Hz.

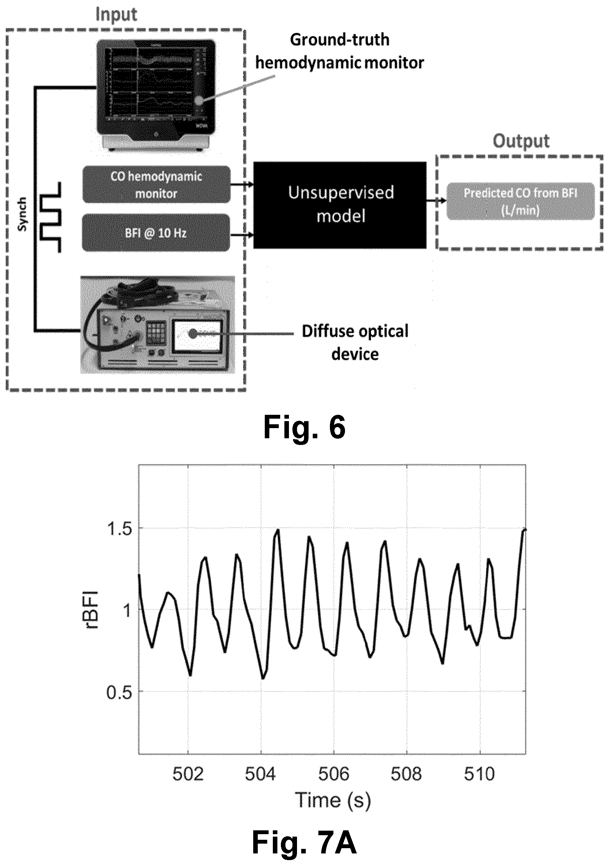

BFI sampled at 10 Hz (Figure 6) was utilized as input of a deep learning model that utilizes leave one-out evaluation and use the temporal and local features to predict the non-invasive CO. A hybrid neural network was implemented by the algorithm.

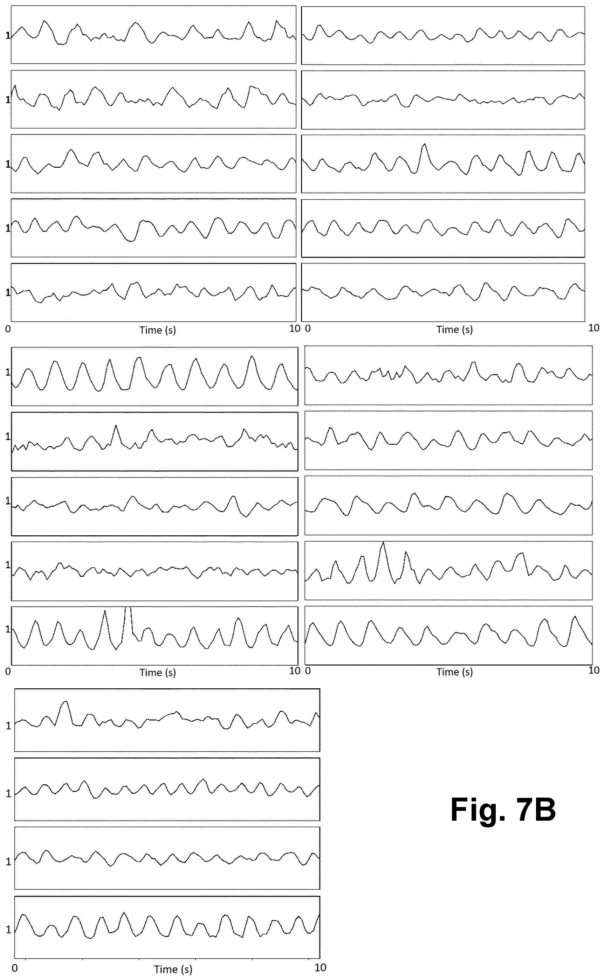

An example of a fast relative BFI (rBFI = BFI(t)/mean(BFI)BSL), where the pulsatile component is present, is shown in Figure 7A, while Figure 7B shows the fast relative BFI for each of the 24 healthy subjects

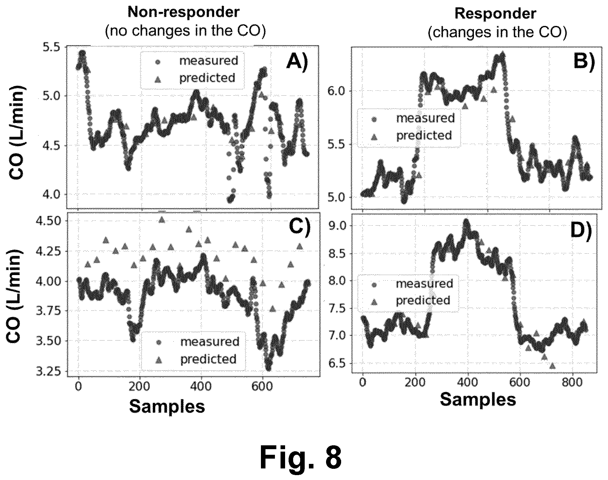

In Figure 8, the present inventors report the example for two non-responder subjects (variation of CO<15% in the PLR period) and two responders (variation of CO>=15%). The solid circles are the CO measured by the hemodynamic monitor (COₘₑₐₛ), while the solid triangles represent the predicted (CO_{pred}) value from the BFI? every 30 samples. The present inventors see that in all the cases the predicted CO values follows the trend of COₘₑₐₛ.

The summary for the root mean square error, bias and standard deviation are reported in Table 1 below.

**Table 1. Summary of root mean square error (RMSE), bias and standard deviation (std) for the two non-responders and two responders.**

| **Subject** | **Type** | **RMSE** | **Bias** | **Std** |
|---|---|---|---|---|
| **A** | Non-responder | 0.23 | -0.01 | 0.15 |
| **B** | Responder | 0.25 | 0.01 | 0.15 |
| **C** | Non-responder | 0.47 | 0.35 | 0.22 |
| **D** | Responder | 0.47 | 0,01 | 0.32 |

### B) Critically ill patients and norepinephrine (NE) dosage:

The previous results (*A)*) demonstrate the feasibility of predicting the CO in healthy subjects where the vascular tone is not compromised, according to the present invention. Here, it is demonstrated that the pulsatile component is still present in subjects admitted to the ICU and with different dosages of norepinephrine (a known first line vasopressor) administered, according to the present invention.

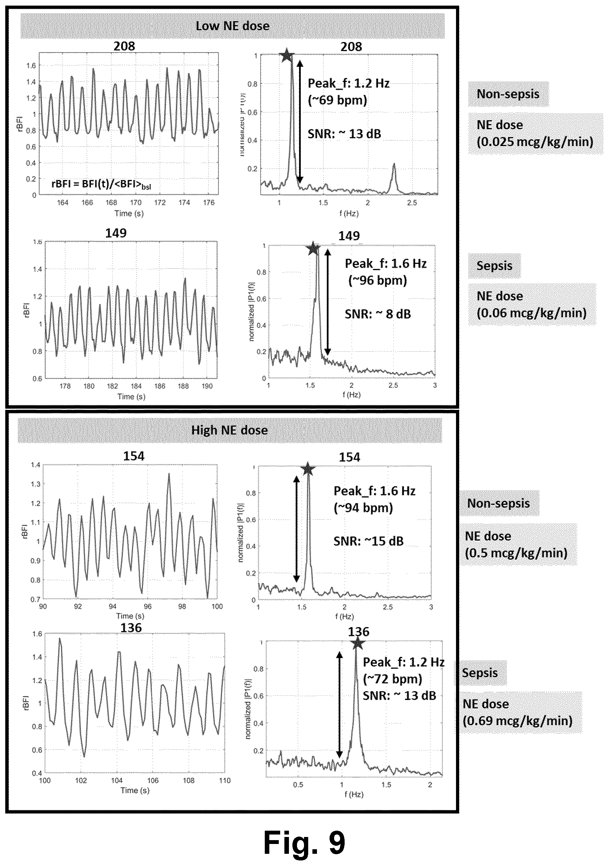

Four patients admitted to the ICU have been selected as an example: two septic patients with low dosage (<= 0.5 mg/kg) of norepinephrine and two non-septic patients with higher dosage of norepinephrine (>0.5 mg/kg). In Figure 9, the patients are reported in a tabular form, with the column representing the fast BFI (at 10 Hz) and the power spectra (obtained through discrete Fourier transform) highlighting the frequency peak (peak_freq) around the heart beat and the signal to noise ratio (SNR in dB). The heart beat reported are comparable with the ones measured by a pulse oximeter integrated in the optical device. As expected, the higher the dosage the higher is the peak frequency, i.e. the heart rate. From these examples, it is possible to observe that with different NE dosages the present inventors are still able to notice a pulsatile pattern in the relative BFI to the baseline. It is possible to use the NE dosage as a further information to be processed to better predict the CO.

### Further applications:

In addition to SV (and thus CO) estimation, the beat-to-beat nature of the measurements will allow for the calculation of additional parameters informing of other crucial physiological qualities like vascular tone, all of them being included for corresponding embodiments of the present invention.

### Vascular tone monitoring:

Vascular tone plays a key role in the relationship between cardiac output and arterial blood pressure. When vascular tone is altered, blood volume will become "unstressed", and a drop in blood pressure will occur. This is the basic mechanism for sepsis, for instance, and having information on vascular tone is fundamental for correctly monitoring and interpreting the hemodynamic status and response to interventions. Indeed, despite an increase in CO as results of a fluid bolus, if vascular tone is altered, no positive response in blood pressure will be observed, leading to misinterpretations of the effects of the hemodynamic intervention. Accordingly, the clinician needs reliable information on vascular tone to complement the resuscitation process, such as adding or modifying vasopressors, for instance. At the bedside, vascular tone can be inferred from dynamic arterial elastance (Eadyn), which has been validated as a useful physiological parameter. Eadyn reflects the relationship between changes in arterial pulse pressure (PP), calculated as systolic and diastolic pressure difference (Psys - Pdias), and SV variations in a beat-to-beat analysis (Eadyn = PPV/SVV) and represents the dynamic interaction between blood pressure (BP) and SV during a respiratory cycle. Therefore it may be useful for the prediction of BP response to fluid challenge in preload-dependent patients, or to predict BP behaviour as results of modifying vasopressor dose. Currently, its monitoring requires continuous measurements of both, cardiac output (CO) and arterial blood pressure. However, as previously exposed, since continuous CO monitoring is performed mainly by PWA technologies, a certain degree of coupling between pressure and the estimated flow is observed in this approach.

Ideally, pressure and flow should be obtained from independent sources. The present invention which implements an optics-based technology will derive, for some embodiments, two different dynamic arterial elastance parameters:
(a) Laser speckle-based technique, such as DCS, derived microvascular bed dynamic elastance (mic-Eadyn). Combining the AUC of the pulsatile signal of the laser speckle-based technique measurements (flow component) with its morphology (pressure component), the present inventors will derive information on the relationship between flow and pressure generation.
(b) When an arterial line is in place, the present invention will combine invasive arterial pressure measurements with the derived microvascular blood flow to estimate a global arterial elastance (glob-Eadyn).

The elastance parameters obtained with the present invention will provide valuable information at the bedside, informing the clinician on the need for adding a vasopressor, or modifying its dose, when an increase in BP is pursued.

A person skilled in the art could introduce changes and modifications in the embodiments described without departing from the scope of the invention as it is defined in the attached claims.

### References:

1. Bootsma IT, Boerma EC, Scheeren TWL, de Lange F. The contemporary pulmonary artery catheter. Part 2: measurements, limitations, and clinical applications. J Clin Monit Comput. 2022;36(1):17-31. doi:10.1007/s10877-021-00673-5.
2. Grensemann J. Cardiac Output Monitoring by Pulse Contour Analysis, the Technical Basics of Less-Invasive Techniques. Front Med. 2018;5:64. doi: 10.3389/fmed.2018.00064.
3. O'Neill R, Dempsey EM, Garvey AA, Schwarz CE. Non-invasive Cardiac Output Monitoring in Neonates. Front Pediatr. 2020;8:614585. doi:10.3389/fped.2020.614585.
4. Berndsen M. Noninvasive and Minimally Invasive Cardiac Output Monitoring: A Nursing Perspective. Dimens Crit Care Nurs DCCN. 2022;41(3):121-123. doi:10.1097/DCC.0000000000000524.
5. Keller M, Magunia H, Rosenberger P, Koeppen M. Echocardiography as a Tool to Assess Cardiac Function in Critical Care-A Review. Diagnostics. 2023;13(5):839. doi:10.3390/diagnostics13050839.
6. Jakovljevic DG, Moore S, Hallsworth K, Fattakhova G, Thoma C, Trenell MI. Comparison of cardiac output determined by bioimpedance and bioreactance methods at rest and during exercise. J Clin Monit Comput. 2012;26(2):63-68. doi:10.1007/s10877-012-9334-4.
7. Jakovljevic DG, Trenell MI, MacGowan GA. Bioimpedance and bioreactance methods for monitoring cardiac output. Best Pract Res Clin Anaesthesiol. 2014;28(4):381-394. doi:10.1016/j.bpa.2014.09.003.
8. Mansfield RC, Kaza N, Charalambous A, Milne AC, Sathiyamurthy S, Banerjee J. Cardiac Output Measurement in Neonates and Children Using Noninvasive Electrical Bioimpedance Compared With Standard Methods: A Systematic Review and Meta-Analysis. Crit Care Med. 2022;50(1):126-137. doi:10.1097/CCM.0000000000005144.
9. Meyer S, Todd D, Wright I, Gortner L, Reynolds G. Review article: Non-invasive assessment of cardiac output with portable continuous-wave Doppler ultrasound. Emerg Med Australas EMA. 2008;20(3):201-208. doi:10.1111/j.1742-6723.2008.01078.x.
10. Kouz K, Scheeren TWL, de Backer D, Saugel B. Pulse Wave Analysis to Estimate Cardiac Output. Anesthesiology. 2021;134(1):119-126. doi:10.1097/ALN.0000000000003553.
11. Saugel B, Kouz K, Scheeren TWL, et al. Cardiac output estimation using pulse wave analysis-physiology, algorithms, and technologies: a narrative review. Br J Anaesth. 2021;126(1):67-76. doi:10.1016/j.bja.2020.09.049.
12. Po JR, Tong M, Meeran T, et al. Quantification of Cardiac Output with Phase Contrast Magnetic Resonance Imaging in Patients with Pulmonary Hypertension. J Clin Imaging Sci. 2020;10:26. doi:10.25259/JCIS_36_2020.
13. Fischer MO, Joosten A, Desebbe O, et al. Interchangeability of cardiac output measurements between non-invasive photoplethysmography and bolus thermodilution: A systematic review and individual patient data meta-analysis. Anaesth Crit Care Pain Med. 2020;39(1):75-85. doi:10.1016/j.accpm.2019.05.007
14. Nedel W, Vasconcellos A, Gunsch K, Rigotti Soares P. Accuracy and precision of oscillometric noninvasive blood pressure measurement in critically ill patients: systematic review and meta-analysis. Anaesthesiol Intensive Ther. 2022;54(5):425-431. doi:10.5114/ait.2022.123120.
15. Park SH, Choi YK. Measurement reliability of automated oscillometric blood pressure monitor in the elderly with atrial fibrillation: a systematic review and meta-analysis. Blood Press Monit. 2020;25(1):2-12. doi: 1 0.1097 /M BP. 0000000000000414.
16. Islam SMS, Chow CK, Daryabeygikhotbehsara R, et al. Wearable cuffless blood pressure monitoring devices: a systematic review and meta-analysis. Eur Heart J Digit Health. 2022;3(2):323-337. doi:10.1093/ehjdh/ztac021.
17. Cheng HM, Lang D, Tufanaru C, Pearson A. Measurement accuracy of non-invasively obtained central blood pressure by applanation tonometry: a systematic review and meta-analysis. Int J Cardiol. 2013;167(5):1867-1876. doi:10.1016/j.ijcard.2012.04.155.
18. Lal SK, Mihailidou AS, Cejnar M, Henderson RJ, Jones M, Hunyor SN. Continuous, non-invasive volume-clamp blood pressure: determinants of performance. J Hypertens. 1993;11(12):1413-1422. doi:10.1097/00004872-199312000-00014.
19. Pannier BM, Avolio AP, Hoeks A, Mancia G, Takazawa K. Methods and devices for measuring arterial compliance in humans. Am J Hypertens. 2002;15(8):743-753. doi:10.1016/s0895-7061(02)02962-x.
20. Noon JP. The arterial pulse wave and vascular compliance. Prog Cardiovasc Nurs. 2009;24(2):53-58. doi:10.1111/j.1751-7117.2009.00032.x.
21. King J, Lowery DR. Physiology, Cardiac Output. In: StatPearls. StatPearls Publishing; 2024. Accessed June 18, 2024. http://www.ncbi.nlm.nih.qov/books/NBK470455/.
22. Mirsajadi, Amirali, et al. "Microvascular Autoregulation in Skeletal Muscle Using Near-Infrared Spectroscopy and Derivation of Optimal Mean Arterial Pressure in the ICU: Pilot Study and Comparison With Cerebral Near-Infrared Spectroscopy." Critical Care Explorations 6.7 (2024): e1111.
23. Payne, Stephen. Cerebral autoregulation: control of blood flow in the brain. Vol. 15. Berlin: Springer, 2016. APA.
24. Brassard, Patrice, et al. "Losing the dogmatic view of cerebral autoregulation." Physiological reports 9.15 (2021): e14982.
25. Lassen, Niels A. "Cerebral blood flow and oxygen consumption in man." Physiological reviews 39.2 (1959): 183-238

## Claims

1. A computer-implemented method for determining a global cardiovascular/hemodynamic parameter of a subject, comprising:
a) receiving data representative of non-invasive and optical measurements of microvascular local blood flow of a muscle or tissue of a current subject, previously acquired by optical techniques based on laser speckle statistics, wherein said muscle or tissue is in a non-compartmentalized area and has a limited autoregulatory performance where the local blood flow cannot be isolated from that of the systemic one;
b) processing said data received at a);
c) determining said global cardiovascular/hemodynamic parameter of said current subject or/and an index originating or representative of the global cardiovascular/hemodynamic parameter, based on the result of step b); and
d) providing said determined global cardiovascular/hemodynamic parameter or/and said determined index.

2. The computer-implemented method of claim 1, wherein said steps b) and c) are performed with the proviso that no physiological data of at least the current subject are used.

3. The computer-implemented method of claim 1 or 2, wherein said steps b) and c) are performed with the proviso that no further measurements of at least the current subject are used, apart from said non-invasive and optical microvascular measurements of microvascular local blood flow of said muscle or tissue of step a).

4. The computer-implemented method of any of the previous claims, wherein said global cardiovascular/hemodynamic parameter is at least one of cardiac output, stroke volume, and vascular tone, or a parameter proportional thereto.

5. The computer-implemented method of any of the previous claims, wherein at least said steps b) and c) are performed with an artificial intelligence learning algorithm trained with at least one training dataset of data of signals representative of non-invasive optical measurements of at least local blood flow of a plurality of previous subjects, wherein the global cardiovascular/hemodynamic parameter of the current subject or/and said index is/are determined by estimating its value, or a transient increase and/or decrease thereof, or no change thereof, with a mathematical estimator learned with said artificial intelligence learning algorithm.

6. The computer-implemented method of claim 5, wherein said at least one training dataset comprises at least one first training dataset including data of signal features of pulsatile signals representative of non-invasive and optical measurements of pulsatile microvascular local blood flow of muscles or tissues of a plurality of previous subjects, previously acquired by optical techniques based on laser speckle statistics, wherein said muscles or tissues are located in non-compartmentalized areas and have a limited autoregulatory performance where the local blood flow cannot be isolated from that of the systemic one, and wherein the pulsatility of said pulsatile signals corresponds to the cardiac cycle.

7. The computer-implemented method of claim 6, wherein said signal features include one or more of following features corresponding to systole and diastole periods of the pulsatile signals: maximum and minimum, an area under the curve, a second peak, and a dicrotic point; and/or at least one or more of the following temporal features: time from the foot to the peak of the pulsatile signal, time from the peak to the foot of the next pulsatile signals, temporal width of said pulsatile signal, cycle duration, upstroke time, and peak to notch time.

8. The computer-implemented method of claim 7, wherein said signal features are extracted and/or learned from signals including at least two different time periods.

9. The computer-implemented method of any of the previous claims, wherein at least said steps b) and c) are performed with an artificial intelligence learning algorithm trained with at least one training dataset of data of signals representative of non-invasive and/or invasive measurements not based on laser speckle statistics of a plurality of previous subjects, wherein the global cardiovascular/hemodynamic parameter of the current subject or/and said index is/are determined by estimating its value, or a transient increase and/or decrease thereof, or no change thereof, with a mathematical estimator learned with said artificial intelligence learning algorithm.

10. The computer-implemented method of any of claims 6 to 9, wherein said at least one training dataset further comprises a training dataset including data of at least said cardiovascular/hemodynamic parameter, for said plurality of previous subjects, obtained from measurements not based on laser speckle statistics but synchronized, for each previous subject, with the non-invasive and optical measurements of pulsatile microvascular local blood flow of a muscle or tissue of the corresponding previous subject, wherein said muscle or tissue is located in a non-compartmentalized area and has a limited autoregulatory performance where the local blood flow cannot be isolated from that of the systemic one.

11. The computer-implemented method of any of claims 6 to 10, wherein said at least one training dataset further comprises a training dataset including at least one of the following data related to the plurality of previous subjects: demographic data, anatomical data, pharmacotherapy data, and augmented data.

12. The computer-implemented method of any of claims 5 to 11, further comprising training said artificial intelligence learning algorithm with the at least one training dataset.

13. The computer-implemented method of claim 12 when depending on claim 5 or 11, further comprising, prior to said training, for the plurality of previous subjects:
- a data acquisition step comprising receiving acquired laser speckle statistics and, synchronized for the same previous subject, said data of at least said global cardiovascular/hemodynamic parameter obtained from measurements not based on laser speckle statistics;
- pre-processing said acquired laser speckle statistics to derive pulsatile signals representative of pulsatile blood flow; and
- filtering the derived pulsatile signals to obtain said pulsatile signals of the at least one training dataset.

14. The computer-implemented method of any of the previous claims, wherein at least said steps b) and c) are performed with a logistic-based regressor used to fit an inputted set of extracted features included in the data received at a) to output the global cardiovascular/hemodynamic parameter or/and the index originating or representative of the global cardiovascular/hemodynamic parameter.

15. A system for determining a global cardiovascular/hemodynamic parameter of a subject, comprising a processing device including a processor and a computer-readable medium having encoded thereon computer-executable instructions to cause the processor to execute the computer-implemented method according to any of claims 1 to 14.

16. A computer program, including code instructions that when executed on at least one processor perform the steps of the computer-implemented method of any of claims 1 to 14.
